# EUROPEAN PATENT SPECIFICATION

(11) **EP 1 828 374 B1**
(45) Date of publication and mention of the grant of the patent: **17.11.2010**
(21) Application number: 04806387.9
(22) Date of filing: 21.12.2004
(51) Int. Cl.: C12N 1/14, C12P 17/18

(54) **A NOVEL ENDOPHYTIC CAMPTOTHECIN AND CAMPTOTHECINOID PRODUCING FUNGUS AND PROCESS OF PRODUCING THE SAME**
NEUES ENDOPHYTISCHES CAMPTOTHECIN UND CAMPTOTHECINOID PRODUZIERENDER PILZ SOWIE VERFAHREN ZUR HERSTELLUNG DAVON
NOUVEAU CHAMPIGNON ENDOPHYTE PRODUCTEUR DE CAMPTOTHECINE ET DE CAMPTOTHECINOIDES ET PROCEDE DE PRODUCTION CORRESPONDANT

(43) Date of publication of application: 05.09.2007
(73) Proprietor: Council of Scientific and Industrial Research, New Delhi 110 001 (IN)
(72) Inventor: Puri, Satish Chander, c/o Regional Res. Lab., Jammu 180001 (IN); Verma, Vijeshwar, c/o Regional Res. Lab., Jammu 180001 (IN); Amna, Touseef, c/o Regional Res. Laboratory., Jammu 180001 (IN); Handa, Geeta, c/o Regional Res. Laboratory, Jammu 180001 (IN); Gupta, Vinay, c/o Regional Res. Laboratory, Jammu 180001 (IN); Verma, Neelam, c/o Regional Res. Laboratory, Jammu 180001 (IN); Khajuria, Ravi Kant, c/o Regional Res. Lab., Jammu 180001 (IN); Saxena, Ajit Kumar, c/o Regional Res. Lab., Jammu 180001 (IN); Qazi, Ghulam Nabi, c/o Regional Res. Lab., Jammu 180001 (IN); Spitller, Michael, Inst. of Environmental Research, D-44227 Dortmund (DE)
(74) Representative: Fiussello, Francesco
(86) International application number: PCT/IB2004/004205
(87) International publication number: WO 2006/067535

(56) References cited:
- LORENCE A ET AL: "Camptothecin, over four decades of surprising findings" PHYTOCHEMISTRY, PERGAMON PRESS, GB, vol. 65, no. 20, October 2004 (2004-10), pages 2735-2749, XP004593909 ISSN: 0031-9422
- STIERLE ANDREA ET AL: "Taxol and taxane production by Taxomyces andreanae, an endophytic fungus of Pacific yew" SCIENCE (WASHINGTON D C), vol. 260, no. 5105, 1993, pages 214-216, XP008051228 ISSN: 0036-8075
- R. STONE: "Surprise! A fungus factory for taxol" SCIENCE, vol. 260, 9 April 1993 (1993-04-09), pages 154-155, XP008051227

## Description

### Field of Invention:

The present invention relates to a novel source of microorganism for production of Camptothecin and related camptothecinoids. The invention also discloses its isolation, screening for Camptothecin production, growth, fermentation requirements and chemical analysis of Camptothecin (camptothecinoids).

### Background Information:

Camptothecin-(CPT), {4-(S)-4-ethyl-4-hydroxy-1-H-pyrano},[3,4,6,7]-indolizino-(1,2b) quinoline-3,14 (4H,12H) dione} which is of the chemical structural formula (Fig-1) belongs to a group of anticancer agents with unique mechanism of action of interfering eukaryotic CPT DNA. and minor camptothecinoids have been obtained in high percentage from Indian tree *Nothapodytes foetida* {Govindachari,.T.R. Phytochemistry 11 3529, (1972)} and Japanese species of *Nothapodytes obscura, N. obtusifolia, N. piltosporsides, N. tomentosa and N. collina {*Zhang XI and Bao Juchen et al. CN.1, 045 266 (CI Co7D39 261 12 Sep.1990, C: A. **114** 1647607v 1991)}. Other sources of camptothecinoids are *Ophiorhiza mungos* Linn (Tafur et al. Llyodia 39.261 1976) *O. phumila* (Katajimia M.et.al.Tenner YuKi Kagobotsi Toronkai Yoshishu 26 493 1997) *Ervatamia heyneana* wall (Gunsekra S.P. et.al. J. Nat. Prod. 42. 475 1979) and *Merriliodendeon megacarpus* Helms (Arisawa .M, et al. Planta. med. 430 404 1981). *Nothapodytes foetida* (syn; *Mappia foetida*) commonly called "Kalagur" is a small tree distributed in western peninsula from Konkan southward i.e. Nilgiris, Anamalis, Pullneys, North Kanara and Konkan ghats. The anticancer agent isolated from this plant displays an unique mechanism of action as it inhibits intra-nuclear enzyme topoisomerase-I, that is required for swivelling and relaxation of DNA during molecular events such as replication and transcription involved in DNA replication.
Clinical trials of several molecules of this class of compound are in progress in different countries such as Irinotecan (CPT-11) and Topotecan (TPT) have been approved for the treatment of metastatic colorectal cancer and refractory ovarian cancer respectively and their clinical pharmacology have been thoroughly discussed in recent review {Plumbo. M. et al. J. Chromatogr. B, 764 (2001) 121}. New potent and water soluble derivatives have been synthesised and are also now in clinical studies. It was established from SAR studies that functionalization at position 7, 9, 10 and even 11 is compatible with increased activity as shown by 9-amino-20-(S)-camptothecin (9-AC), CPT-11, TPT, N-desmetyl-TPT and other amino containing moieties "Exatecan" (DX-8951) (Mitsui, I.. et al, Jpn. J.Cancer Res 86 (1995) 776). Second generation potent drug candidates at the preclinical stage are more active than CPT like lurtotecan (GG211 or G-1147211) piperazinyl derivative with five or six-member ring at position 10 and 11 which exhibits anti-leukaemia and anti-tumour activity (Takimoto. C.H. et al, Biochim. Biophys. Acta, 1400(1998) 107).
The renewable wild source of *Nothapodytes foetida* has experienced a colossal depletion due to rapidly disturbed and altered eco-system. The germination of *Nothapodyte's* seeds is tedious due to compressed dormancy.
Even though CPT has shown remarkable promise as an anti-tumor agent, unfortunately at the present time the supplies of CPT are inadequate when compared to its projected demand. Thus it is essential to understand how, where and when CPT is biosynthesised in the plants and factor that affect its biosynthesis. As it is well known that many factors influence the production of bioactive molecules which not only include various environmental factors such as temperature and moisture level, but the genetic background of the tree itself: Also plants are commonly hosts to a multitude of microbes including parasites, symbionts, endophytes, epiphytes and mycorrhizal fungi. These organisms may also influence the production of secondary plant metabolites such as phytoalexins whose presence can be triggered by elicitors from microbes. Such microbes may also be capable of production of secondary molecules similar to those produced by the plants.
There are other reasons that prompted the present inventors to devise an *"in-vitro"* system of CPT production. The system can utilises precursors of CPT, an optimised environment and appropriate plant parts where it is synthesised. The results will be an *"in-vitro"* system for CPT from the most productive aerial tissue portions of indigenous *Nothapodytes.*
However such "*in-vitro*"synthesis has many limitations. The source of CPT production from the wild sources either from Chinese *Camptotheca accuminata* or *Nothapodytes foetida* is relatively difficult to meet the demand of the parent compound CPT in view of therapeutic applications in anticancer therapy. Presently isolation from the plant sources or synthesis of camptothecinoids involves multi-step procedure and will be uneconomic.

In Phytochemistry, vol 65, no. 20 (2004-10), pp. 2735-2749, Lorence et al. disclosed the isolation of camptothecin from several different plant species. In their paper, explicit reference was also made to the wish for other sources for camptothecin.

Although Stierle et al taught in Science, vol. 260, no. 5105 (1993), pp. 214-216 to produce the anti-tumour alkaloid taxol from the bark of a tree, thus suggesting that alternative sources for a molecule might be sought in that type of material, no such result has been achieved for camptothecin in over 10 years from such breaking publication, as also commented by Stone in Science, vol 260, (1993-04-09), pp. 154-155.

**It is therefore, the objective of the present inventors to investigate, identify and isolate a novel organism for the production of CPT. It is also an object of the present invention to use this microbial source and utilise the fermentation and biotechnology capabilities as a novel mode of preparation of such molecules.**

In order to meet the gap between demand and supply of these resources and reduce the greater reliance on wild resources, it is not possible to wait for natural regeneration of vegetative biomass. Therefore attempts have been made to find alternative source in shortest possible time for production camptothecinoids.

### Objects of the invention:

The main object of the present invention relates to a novel endophytic fungal strain having accession no. MTCC 5124, deposited at International Depository at Institute of Microbial technology (IMTECH), Chandigarh, New Delhi, for production of Camptothecin and Camptothecinioids (CPT).

Another object of the present invention relates to a process of producing and isolating Camptothecin and camptothenoids from a novel endophytic fungal strain having accession no. MTCC 5124, deposited at International Depository at Institute of Microbial technology (IMTECH), Chandigarh, New Delhi, for production of Camptothecin and Camptothecinioids (CPT)

Yet another process is described which relates to a process of isolating a novel endophytic fungal strain having accession no. MTCC 5124, deposited at International Depository at Institute of Microbial technology (IMTECH), Chandigarh, New Delhi, for production of Camptothecin and Camptothecinioids (CPT).

### Brief description of Accompanying Drawings/Figures:

- Fig.1: (a) Microscopic view of horizontally growing unbranched stoloniferous hyphae (Mag. × 500)
(b) Microscopic view of horizontally growing unbranched stoloniferous hyphae (Mag. × 1000)
(c) Microscopic view of the young sporangium of endophytic fungus (Mag. × 1000)
- Fig. 2: HPLC profile of (a) authentic camptothecin; (b) Fungal Camptothecin and (c) Cospiked of a & b
- Fig. 3: (a) LC/MS profile of authentic camptothecin
(b) LC/MS profile of fungal camptothecin

### Summary of the Invention:

The present provide a new source in form of a novel endophytic fungal strain having accession no. MTCC 5124, deposited at International Depository at Institute of Microbial technology (IMTECH), Chandigarh, New Delhi, for production of Camptothecin and Camptothecinioids (CPT) and an improved process for producing the same. The present investigation has provided a new source and use fungi for the production of CPT. The fungi has been isolated from the inner bark of a specific *Mappia* species belonging to northwestern Himalayan agro-climatic region.

### Description of the Invention:

In terrestrial life microbes exist as parasites, saprophytes endophytes, symbionts or mycorrhizae with possible intergeneric exchange of genetic information between them and their hosts or vice versa. It is well documented that few agrobacteria are capable of genetically transforming their host plants via either the Ri or Ti plasmids which results the formation of genetically transformed plant having one or more characteristics of agrobacterium. Phytohormones such as indole acetic acid, gibberllins, cytokinnins or abscissic acid, can be produced in the plant kingdom by one more plant parasitic bacteria or fungi.

Thus, there is no published evidence that CPT could or would be produced by any micro-organism, the present inventors explored the micro-organism option- the rationale being, that CPT (camptothecinoids) may be produced by one or more microbes associated with the *Mappia* tree.

Logically the basis for this concept is that microbes exists which will produce CPT due genetic exchange previously occurred, either between the microbe(s) (as original source of CPT ) or *Mappia* (as original source of CPT). The net result would be the most desirable case of possessing one or more microbes which could be placed in fermentors to produce 20(S) - CPT or other camptothecinoids.

The present invention provides a new source and improved process for producing CPT and its congeners, wherein the investigators have identified a novel endophytic fungal strain having accession no. MTCC 5124, deposited at International Depository at Institute of Microbial technology (IMTECH), Chandigarh, New Delhi, for production of Camptothecin and Camptothecinioids (CPT),

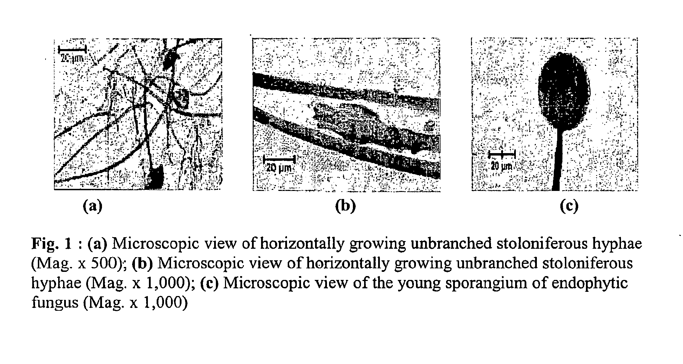

This fungus is an endophytic fungus having small hyphae which average 2-3 µm in diameter the mycelia are branched, aseptate, ribbon shaped and multinucleate. Sporangiophores are long, unbranched, and wide and terminate into a sporangium oval to round in shape **(****Fig. 1****).** This fungus grows rapidly on many common media, covering plates with its mycelium in 7 to 8 days. The said fungi was obtained from the inner bark of a specific *Mappia* species belonging to northwestern Himalayan agro-climatic region.

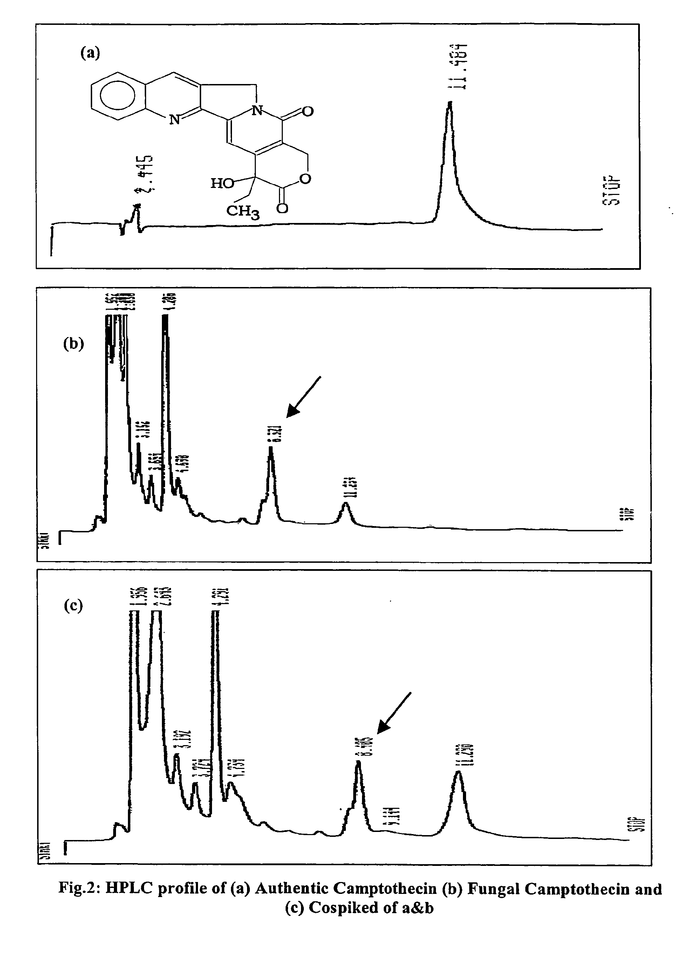

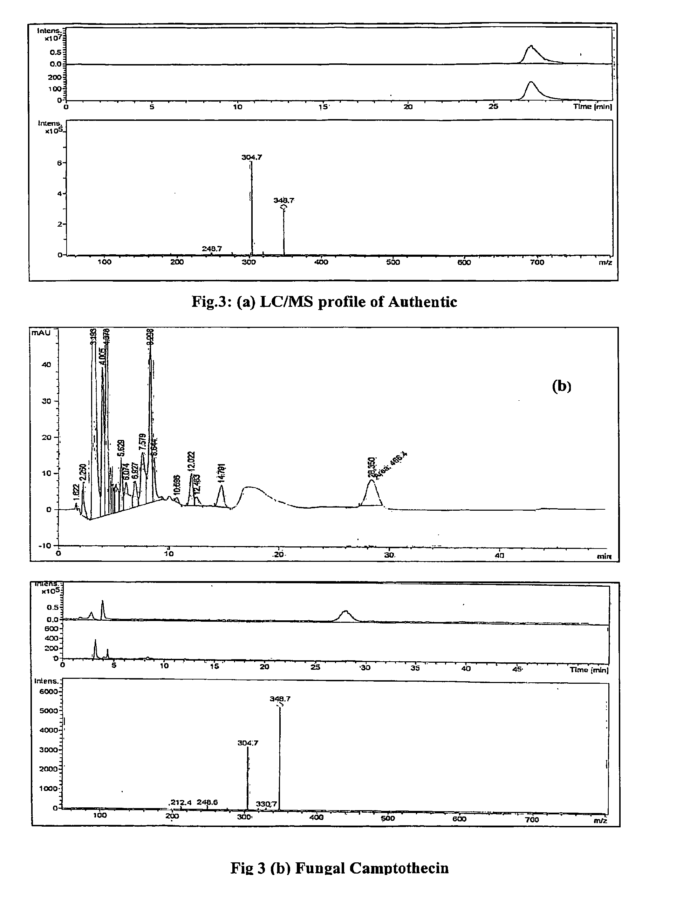

The fungus was isolated and identified by placing said tissue fragments on agar medium for 2-7days and isolating the culture-an endophyte from inner stem bark fragments after surface sterilisation of bark fragment with 50-70% ethanol for 1-20 minutes. Thereafter the fungal hyphae from the said agar medium on mycological agar and again placing said fungal hyphae on said mycological agar if necessary, until a culture in pure form is obtained. extracting all three fractions i.e. cytosolic material, residual pellets and supernatant with solvent as described in (F) above, 3-4 times and evaporating the solvent. The further studies involves to test the production of CPT from the fungi using HPLC and LC/MS and MS-MS studies (Fig. 2 and 3).

The present study also involved the total DNA isolation from the mucelia of the fungus using modified method as described by Vainio *et al* (1998). Using the MICRO SEQ D2, using fungal sequencing kit ABI (ABI, USA) the large subunit (LSU) r DNA of the fungus was identified. The present description further discloses a class of microbes which have natural (20S) - CPT producing characteristics, the details of which are described herein and is only a representative of such microbes and may not be restricted to it alone. The endophytic microbes isolated according to the present procedure produces CPT when cultured under fermentation conditions also discussed herein. - Thus invention provides a fungus which has an ability for the production of CPT.

In the present invention the inventors have also provided studies for improved method for producing a bulk pharmaceutical composition, which contains a pharmaceutically effective amount of a CPT composition, combined with one or more pharmaceutically acceptable inert or physiologically active diluents or adjuvant.

Moreover, the present invention includes a novel method for the production of an agent for the treatment of gastric cancer, intestinal, head and neck tumours and bladder carcinoma.

The present description is directed to fungi, which have camptothecinoids producing characteristics especially as described in the present invention irrespective of their sources. Such microbes are those which can produce CPT authenticated by HPLC, LC/MS and LC/MS/MS. Characteristic ions m/z349(M+H⁺), m/z 305, 275, 249, 248, 220, 219, 206 and 168 were identical with those of the authentic CPT.

Accordingly, the experiments clearly show that the CPT is being synthesised by the microbe in a given medium.

Accordingly, the main embodiment of the present invention relates to a novel endophytic fungal strain having accession no. MTCC 5124, deposited at International Depository at Institute of Microbial technology (IMTECH), Chandigarh, New Delhi, for production of Camptothecin and Camptothecinioids (CPT), said strain has following characteristics:
(a) fungal strain forms white colony mycelium and turns black during sporulation,
(b) forms aerial hyphae, which are round to globose and terminal.

Another embodiment of the present invention relates to the fungal strain, wherein the fungal strain has been isolated from bark of tree *Nothapodytes foetida.*

Another embodiment of the present invention relates to fungal strain, wherein said strain produces about 25 µg/mg of CPT.

Another embodiment of the present invention relates to fungal strain, wherein said strain produces about 18 µg/mg of CPT.

Another embodiment of the present invention relates to fungal strain, wherein said strain produces CPT within 6-7 days of culturing.

Another embodiment of the present invention relates to a new use of novel endophytic fungal strain having accession no. MTCC 5124, deposited at International Depository at Institute of Microbial technology (IMTECH), Chandigarh, New Delhi, for production of Camptothecin and Camptothecinioids (CPT), said strain has following characteristics:
(a) fungal strain forms white colony mycelium and turns black during sporulation,
(b) forms aerial hyphae, which are round to globose and terminal.

Still another embodiment of the present invention relates to a process of isolating Camptothecin and Camptothecinioids (CPT) from novel endophytic fungal strain having accession no. MTCC 5124, said process comprising the steps of:
(a) incubating the fungal strain on to a carbohydrate medium for about 6-7 days at temperature of 28 ± 2°C at 220-220 rpm,
(b) filtering the fungal mycelia through whatman paper under vacuum to obtain the fungal pellet,
(c) washing the fungal pellet obtained in step (b) with Tris-HCl buffer (pH-6 to 7),
(d) resuspending the washed fungal pellet in the same buffer in the ratio of 1:4,
(e) homogenizing and centrifuging the resuspended pellet at 4°C at 10,000 to 12, 000 rpm for 15-30 minutes,
(f) extracting the supernant of using organic solvents, and
(g) identifying CPT using conventional means

Yet another embodiment of the present invention relates to a carbohydrate medium, wherein carbohydrate medium in step (a) consist of Peptone about 10 g/litre, Dextrose 40 g/l, Agar

One more embodiment of the present invention relates to the organic solvents, wherein organic solvents in step (f) are selected consist of chloroform and methanol mixture in the ratio of 4:1.

Another embodiment of the present invention relates to conventional methods, wherein step (g) the conventional method for identifying CPT involves HPLC, LC/MS.

One more mebodiment of the present disclosure relates to a process of isolating a novel endophytic fungal strain having accession no. MTCC 5124 from bark of tree *Nothapodytes foetida* for production of Camptothecin and Camptothecinioids (CPT), said process comprising steps of:
(a) cutting small stems from tree of *Nothapodytes foetida ,*
(b) disinfecting the stems with ethanol (5-70%),
(c) removing the outer bark using a sterile blade,
(d) putting the pieces of inner bark of the stem onto a agar medium
(e) achieving the growth of endophytic fungus in 2-5 days,
(f) reculturing the fungus onto a fresh PDA medium for growth and multiplication, and
(g) identifying the characteristics of the fungal strain.

The present invention thus provides a new source for the production of the CPT. The earlier known sources for the CPT's were highly expensive and time consuming thus being highly uneconomical. The new source identified in the present study is non-obvious to the person skilled in the art as it's the first source of its kind to be identified which produces CPT. Since CPT is in great demand due to his application in medicine as anticancer compound, therefore it is a necessity and imperative to use and identify alternate sources for CPT production, which are cheaper and economically viable. Further, the presently known source i.e. *Nothapodytes foetida* is highly endangered plant species and has a very fragile ecosystem. As there is a limited resource of this plant therefore a person skilled in the art and the filed of medicine will appreciate and value such invention and study. Although various investigators have looked for the alternate resources for producing CPT but have not been very successful. No doubt tissue culture technique have been employed in the culturing and multiplying the plants of *Nothapodytes foetida* for CPT production. However, this technique is also very tedious as this plant is highly recalcitrant. Therefore identification of a new source which is non-recalcitrant would be of great benefit.

The detailed description illustrates the invention by way of example.

### EXAMPLES

### Example-1

The microbe is a novel endophytic fungus (MTTC 5124) associated with inner bark of *Nothapodytes foetida* (Wight). This microbe was obtained from the inner bark of a specific *Mappia* species belonging to north-western Himalayan agro-climatic region but may not restrict to this species alone.

The aerial tissue fragments from a fully grown tree or the seeds thereof of the genus *Nothapodytes foetida a* medicinal plant which grows widely throughout India including in the North-western Himalayan agro climatic region as well as from southern Konkan forests. The medicinal plant's best suited habitat of north-western agro climatic region is commonly found in north-western ghats of India.

This tissue fragments were placed on agar medium for 2-7days and isolating the culture-an endophyte from inner stem bark fragments after surface sterilisation of bark fragment with 50-70% ethanol for 1-20 minutes.

A fungal culture designated as (MTTC 5124) was isolated from inner bark of *Mappia* tree and grown on peptone dextrose broth (PDA, peptone 10, dextrose 40 gl⁻¹, pH 5-6) and incubated at 28 ± 2°C in an incubator shaker (200-220 rpm) for 6-7 days. The fungus grows into a mass of mycelium and sporulates sparsely during incubation. The mycelial mat along with the spores was filtered through the Whatman filter paper under vacuum till all the broth was removed. The pellet was washed with Tris.HCl (pH 6-7) buffer and resuspended in the same buffer in the ratio of 1:4. The mycelial mat was homogenized in a homogenizer and centrifuged at 4°C, 10,000-12,000 rpm for 15-30 min. The supernatant was extracted with chloroform and methanol (4:1) mixture. To further confirm the presence of CPT on HPLC and was found identical with authentic control. The left over residue from centrifuged sample was also examined for CPT by HPLC and was also found to contain CPT in traces **(****Fig. 2** **a,b,c ;** **Fig. 3a and 3b****).** This was further checked with UV inspection chamber when CPT gave blue spots on comparison with one or more camptothecinoids standards such as CPT, 9-methoxycamptothecin and Mappacin. The characteristic ions m/z349(M+H⁺), m/z 305, 275, 249, 248, 220, 219, 206 and 168 were identical with those of the authentic CPT.

### Example- 2

Agar blocks having fungus (MTTC 5124) were fixed and dehydrated for transmission and scanning electron microscopy (SEM). The drying procedure caused some shrinkage of biological structures which means they would be slightly larger and the clumps of cells more tightly packed in the living state **(****Fig. 1****).**

### Example-3

### Cultural characterisation of fungal culture

When an agar plug of inoculum of fungal culture was placed in the centre of freshly prepared agar plates enriched with various nutrients, it grows rapidly and reaches the edge of the plate in 4-5 days. Numerous fluffy aerial mycelial were observed on the agar and the fungus sporulated after 6-7 days with blackish grey coloration.

### Example-4

A fungal isolate designated as (MTCC 5124) was grown on medium as described in Example 1. The extracted CPT was co-spiked with standard CPT for authentication of the molecule. The extracted CPT and the added authentic CPT appeared together on the chromatogram thereby confirming the presence of CPT in the culture extract.

The TLC studies carried out showed an A band at R_{f} 0.67-0.70 that showed blue absorbance at 254 nm. was scraped from the plate and eluted with acetonitirile. This band had same FAB mass spectrum as authentic CPT with an molecular ion peak at m/z 348.7 and prominent peaks at m/z 319.6, 304, 291,289, 276, 248 219 205, 191,140 and 109.

The UV spectrum of the compound has an absorbance maximum which is identical to authentic CPT.

In addition the Rf values of CPT preparation from fungus was identical to be authentic CPT.

The amount of CPT (6-7days culture) is estimated up to 18 µg/mg of chloroform extract.

The taxonomy and properties of the microbes revealed to be a fungus belonging to family phycomycetes.

## Claims

1. Endophytic fungal strain having accession no. MTCC 5124, deposited at International Depository at Institute of Microbial technology (IMTECH), Chandigarh, New Delhi, for the production of Camptothecin and Camptothecinioids (CPT), said strain having the following characteristics:
(a) fungal strain forms white colony mycelium and turns black during sporulation, and
(b) forms aerial hyphae, which are round to globose and terminal.

2. A process of isolating Camptothecin and Camptothecinioids (CPT) from endophytic fungal strain having accession no. MTCC 5124, said process comprising the steps of:
a. incubating the fungal strain on a carbohydrate medium for about 6-7 days at temperature of 28 ± 2°C at 200-220 rpm,
b. filtering the fungal mycelia through Whatman paper under vacuum to obtain the fungal pellet,
c. washing the fungal pellet obtained in step (b) with Tris-HCl buffer (pH-6 to 7),
d. resuspending the washed fungal pellet in the same buffer in a 1:4 ratio,
e. homogenizing and centrifuging the resuspended pellet at 4°C at 10,000 to 12,000 rpm for 15-30 minutes,
f. extracting the supernatant of the homogenized and centrifuged suspension using organic solvents, and
g. identifying CPT using conventional means.

3. A process as claimed in claim 2, wherein the carbohydrate medium in step (a) consists of Peptone about 10 g/l, Dextrose 40 g/l, Agar.

4. A process as claimed in claim 2, wherein the organic solvents used in step (f) consist of a mixture of chloroform and methanol in the ratio of 4:1.

5. A process as claimed in claim 2, wherein the conventional means for identifying CPT of step involve (g) HPLC, LC/MS.

6. A process as claimed in claim 2, wherein said strain produces about 25 µg of CPT per mg of fungal strain.

7. A process as claimed in claim 2, wherein said strain produces about 18 µg of CPT per mg of fungal strain.

## Patentansprüche

1. Endophytischer Pilzstamm mit der Hinterlegungsnummer MTCC 5124, hinterlegt bei der Internationalen Hinterlegungsstelle am Institute of Microbial Technology (IMTECH), Chandigarh, Neu-Delhi, für die Herstellung von Camptothecin und Camptothecinoiden (CPT), wobei der Stamm die folgenden Eigenschaften besitzt:
(a) der Pilzstamm bildet Kolonien mit einem weißen Myzel und verfärbt sich schwarz während der Sporulierung, und
(b) der Pilzstamm bildet Lufthyphen, welche rund bis kugelförmig und endständig sind.

2. Verfahren zur Isolierung von Camptothecin und Camptothecinoiden (CPT) aus dem endophytischen Pilzstamm mit der Hinterlegungsnummer MTCC 5124, wobei das Verfahren die folgenden Schritte umfasst:
(a) Inkubieren des Pilzstamms in einem Kohlenhydratmedium für etwa 6-7 Tage bei einer Temperatur von 28 ± 2°C bei 200-220 UpM;
(b) Abfiltrieren des Pilzmyzels durch Whatman-Filterpapier unter vermindertem Druck, um ein Pilzpellet zu erhalten;
(c) Waschen des in Schritt (b) erhaltenen Pilzpellets mit Tris-HCl-Puffer (pH 6 bis 7);
(d) Resuspendieren des gewaschenen Pilzpellets in demselben Puffer in einem Verhältnis von 1:4;
(e) Homogenisieren und Zentrifugieren des resuspendierten Pellets bei 4°C bei 10.000 bis 12.000 UpM für 15-30 Minuten;
(f) Extrahieren des Überstands der homogenisierten und zentrifugierten Suspension unter Verwendung von organischen Lösungsmitteln; und
(g) Identifizieren von CPT mit Hilfe von herkömmlichen Mitteln.

3. Verfahren wie in Anspruch 2 beansprucht, wobei das Kohlenhydratmedium in Schritt (a) aus etwa 10 g/l Pepton, 40 g/l Dextrose und Agar besteht.

4. Verfahren wie in Anspruch 2 beansprucht, wobei die in Schritt (f) verwendeten organischen Lösungsmittel ein Gemisch aus Chloroform und Methanol in einem Verhältnis von 4:1 umfassen.

5. Verfahren wie in Anspruch 2 beansprucht, wobei die herkömmlichen Mittel für die Identifizierung von CPT in Schritt (g) eine HPLC oder eine LC/MS umfassen.

6. Verfahren wie in Anspruch 2 beansprucht, wobei der Stamm etwa 25 µg CPT pro mg des Pilzstamms produziert.

7. Verfahren wie in Anspruch 2 beansprucht, wobei der Stamm etwa 18 µg CPT pro mg des Pilzstamms produziert.

## Revendications

1. Souche de champignon endophyte ayant le numéro d'accession MTCC 5124, déposée auprès de l'Autorité de Dépôt Internationale (International Depositary) de l'Institut de technologie microbienne (Institute of Microbial Technology) (IMTECH), Chandigarh, New Delhi, pour la production de camptothécine et de camptothécinoïdes (CPT), ladite souche ayant les caractéristiques suivantes :
(a) la souche fongique forme un mycélium de colonie blanc et devient noire pendant la sporulation, et
(b) forme des hyphes aériens, qui sont ronds à globuleux et terminaux.

2. Procédé d'isolement de camptothécine et de camptothécinoïdes (CPT) à partir de la souche fongique endophyte ayant le numéro d'accession MTCC 5124, ledit procédé comprenant les étapes consistent :
a. à incuber la souche fongique sur un milieu glucidique pendant environ 6 à 7 jours à une température de 28 ± 2 °C à 200 - 220 tr/min.,
b. à filtrer les mycéliums fongiques à travers du papier Whatman sous vide pour obtenir le granule fongique,
c. à laver le granule fongique obtenu dans l'étape (b) avec du tampon Tris-HCl (pH 6 à 7),
d. à remettre en suspension le granule fongique lavé dans le même tampon dans un rapport 1:4,
e. à homogénéiser et centrifuger le granule remis en suspension à 4 °C à 10 000 à 12 000 tr/min. pendant 15 - 30 minutes,
f. à extraire le surnageant de la suspension homogénéisée et centrifugée en utilisant des solvants organiques, et
g. à identifier les CPT en utilisant des moyens classiques.

3. Procédé selon la revendication 2, dans lequel le milieu glucidique dans l'étape (a) consiste en peptone à environ 10 g/l, dextrose à 40 g/l, gélose.

4. Procédé selon la revendication 2, dans lequel les solvants organiques utilisés dans l'étape (f) consistent en un mélange de chloroforme et de méthanol dans le rapport de 4:1.

5. Procédé selon la revendication 2, dans lequel les moyens classiques pour identifier les CPT de l'étape (g) comprennent la CLHP, la CL/SM.

6. Procédé selon la revendication 2, dans lequel ladite souche produit environ 25 µg de CPT par mg de souche fongique.

7. Procédé selon la revendication 2, dans lequel ladite souche produit environ 18 µg de CPT par mg de souche fongique.
